# EUROPEAN PATENT APPLICATION

(11) **EP 3 070 175 A1**
(43) Date of publication of application: **21.09.2016**
(21) Application number: 15305409.3
(22) Date of filing: 20.03.2015
(51) Int. Cl.: C12Q 1/18, C12Q 1/04

(54) **TEST FOR DETERMINING SUSCEPTIBILITY OR RESISTANCE TO POLYMYXINS IN ENTEROBACTERIACEAE**

(71) Applicant: Université de Fribourg, 1700 Fribourg (CH)
(72) Inventor: Nordmann, Patrice, 1700 Fribourg (CH); Poirel, Laurent, 1700 Fribourg (CH); Jayol, Aurélie, 1700 Fribourg (CH)
(74) Representative: Cabinet Plasseraud

(57) **Abstract**

Method for determining in solution susceptibility or resistance to a polymyxin antibiotic of bacteria of the *Enterobacteriaceae* family, comprising the steps consisting in
a) providing a liquid composition comprising
- a liquid growth medium for *Enterobacteriaceae,*
- a polymyxin antibiotic compound at a defined concentration adapted to inhibit *Enterobacteriaceae* sensitive to polymyxin antibiotics,
- a pH indicator providing a colour to the liquid composition,
- a substrate comprising one or more aldohexoses,
b) providing into the liquid composition a biological sample containing one or more bacteria of the *Enterobacteriaceae* family,
c) carrying out a metabolization reaction of glucose,
d) watching a colour change of the liquid composition
and diagnostic kit for implementing the method.

## Description

The present invention relates to a test and kit for determining susceptibility or resistance to polymyxins in *Enterobacteriaceae.*

Multi-drug resistance (MDR) to antibiotic in bacteria is becoming now a major issue worldwide. Those bacteria account for most hospital infections worldwide (eg in the USA affecting 2 million people and causing 25,000 deaths annually). Mortality rates from sepsis in intensive care units range from 20 to 60% worldwide, and one retrospective study revealed that 20% of patients with septic shock were initially treated with inappropriate antibiotic therapy. This is largely because its takes days to obtain a diagnostic of pathogens and their susceptibility patterns. A study reports that the risk for in-hospital mortality increased by 9% for every hour of delay before the correct antibiotic regimen is administered. Thus, the rapidness of identification of the pathogen and its susceptibility pattern can mean difference between life and death in severe infections such as bloodstream infections.

Many important public bodies have already raised an alarm signal on the rapid and uncontrollable spread of MDR bacteria such as the WHO, the European Center for Control Diseases in Stockholm and the Center for Diseases control (CDC) in Atlanta in the USA and most recently the International Monetary Fund and the White House in the USA. A multiple facet approach for combating multidrug resistance is now evidenced. Basically, this strategy is intended to reach four synergistic goals (i) antibiotic stewardship from agriculture to human medicine, (ii) surveying emerged and emerging resistance determinants, (iii) accelerate basic and applied research leading to the development of novel antibiotics, other therapeutics and vaccines development and (iv) use of rapid and innovative diagnostic tests for identification and characterization of antibioticresistant bacteria.

The most important sources of concern in MDR are focused in *Enterobacteriaceae* such as *Escherichia coli* and *Klebsiella pneumoniae* which are the main sources of the most infections in humans (both community- and hospital-acquired infections). Those infections are mostly urinary tract infections, bloodstream infections, pulmonary and intra-abdominal infections. Their treatment mostly relies on ß-lactams such as penicillins, cephalosporins, the broadest spectrum ß-lactams i.e. carbapenems, aminoglycosides and quinolones. Acquired resistance to those antibiotic molecules is increasingly reported worldwide, being often associated to MDR bacteria. Very few (if any) therapeutic options are left. Therefore, a renewed interest in an old class of antibiotics, polymyxins, colistin and polymyxin B, is observed worldwide. As a recent example, large use of polymyxins is now observed for treating bloodstream infections due to *Klebsiella pneumoniae* in Italy which show a very prevalence rates (40-60%) of carbapenem resistance is reported due to the spread of carbapenemase producers. This large use of polymyxins in this country explains why colistin resistance superimposes to carbapenem resistance in *Enterobacteriaceae.*

Polymyxins became clinically available in the 1950's but soon fell out of favour mainly because of concerns of their potential toxic effects in the kidneys and development of plenty of efficient and much less toxic antibiotics (cephalosporins, carbapenems, aminoglycosides, fluoroquinolones). Polymyxin molecules were mostly used only in veterinary medicine until recently and as attempts to decolonize patients carrying MDR bacteria.

As products of bacterial fermentation (from *Bacillus polymyxa* subpecies *colistinus),* colistin and polymyxin B are multicomponent antibiotics active only against several gram-negative species. They have very similar chemical structures, differing by one amino acid in the peptide ring. The polymyxins are relatively large lipopeptide molecules and are poorly absorbed after oral administration. For the treatment of life-threatening systemic infections, they are given by the intravenous route or by nebulisation for the treatment of respiratory tract infections. Likewise other antimicrobial peptides, colistin interacts with the lipid A moiety of the lipopolysaccharide (LPS) of gram-negatives. The polycationic peptide ring competes for and substitutes the calcium and magnesium bridges stabilizing the LPS, promoting membrane permeability and thus disrupting the integrity of the outer membrane of Gram negatives, thus leading to bacterial death. Although polymyxin resistance rates are relatively low in developed countries (except in Italy, Spain and Greece), there is a concern that this situation might be changing rapidly. The main mechanisms of resistance to polymyxins in *Enterobacteriaceae,* in particular in *K. pneumoniae,* are modifications in genes modifying the LPS leading to a weak (if any) binding of polymyxins to the outer-membrane proteins.

In this area of growing use of polymyxins in human medicine, there is an increasing need for testing polymyxins susceptibility and resistance rapidly.

Several techniques have been proposed to determine in-vitro susceptibility to polymyxins. Disk diffusion technique (a paper disk containing a polymyxin molecule diffusing in an agar gelose) and the E-test technique (a plastic strip with a predetermined gradient of polymyxin concentration diffusing in an agar gelose) have been proposed to determine those polymyxins minimum inhibitory concentrations (MICs). Corresponding results are obtained within 18 h. Those techniques are not reliable, as for an example the E-test technique may be the source of 9-32% very major errors as compared to the reference technique that is the broth dilution technique. The broth dilution technique consists in putting 1log2 dilution of polymyxin concentrations in a cation-adjusted broth medium in presence of an inoculum of ca. 10⁴ colony forming units per ml of the bacterial strain to be tested. Results of susceptibility or resistance are obtained in 18 h by comparing those obtained MIC values to the breakpoint values as determined by the EUCAST (European Committee on Antimicrobial Susceptibility testing from the European Society for Microbiology and Infectious Diseases) recommendation for colistin (susceptibility ≤ 2 mg/L and resistance > 2 mg/L). Each time MIC values of polymyxins are needed for treating patients with polymyxins using home-made dilution technique, preparation of the culture media, powder weighting etc. which is time-consuming. MICs values have to be determined specifically for each polymyxin clinically used (polymyxin B and colistin) since extrapolation of susceptibility for polymyxin B and colistin cannot be made. A research-use only commercial test is available in the USA for determining polymyxin MICs (TREK Diagnostic Systems, Inc. Cleveland, Oh.) This test is adapted to test a series of isolates rather than single isolates. This research-use only labelling indicates that this test shall not be used for patient testing and results are available in 16- 18 h

Molecular-based techniques for identification of polymyxin resistance cannot be envisioned in a near future since we are only at the edge of unravelling the mechanisms of this resistance trait in *Enterobacteriaceae.* Many of the polymyxin resistance mechanisms result from point mutations in regulatory genes requiring the need not only of PCR amplification but also sequencing results. It is possible that in many clinical isolates phenotypic resistance to polymyxins corresponds to a multiplicity of associated resistance mechanisms in single isolates (for example defect in outer-membrane proteins combined to structural modification of the LPS). In addition, an intrinsic limitation of all genotypic methods is that they detect potential for resistance whereas phenotypic methods detect the realization of susceptibility (i.e. no growth in the presence of antibiotics,) that is far more relevant as a basis for a therapeutic decision.

Therefore a rapid diagnostic test for determining susceptibility or resistance to polymyxins is needed for its daily use in clinical settings.

It is an object of the present invention to eliminate or at least to substantially mitigate said drawbacks of existing methods of identification of polymyxin susceptibility or resistance.

The present invention is based on the colorimetric detection of growth of bacteria of the Enterobacteriaceae family in relation with aldohexose (for example glucose, mannose or arabinose) metabolization (oxydation and fermentation), in presence of a defined concentration of a polymyxin antibiotic. A rapid metabolization of aldohexoses reveals bacterial growth in presence of a polymyxin antibiotic, i.e. resistance to polymyxin. A change of pH may be evidenced by using a pH indicator since metabolization of aldohexoses provides acid metabolites.

A subject of the present application is therefore a method for determining in solution susceptibility or resistance to a polymyxin antibiotic of bacteria of the *Enterobacteriaceae* family, comprising the steps consisting in
a) providing a liquid composition comprising
   - a liquid growth medium for *Enterobacteriaceae,*
   - a polymyxin antibiotic compound at a defined concentration adapted to inhibit *Enterobacteriaceae* sensitive to polymyxin antibiotics,
   - a pH indicator providing a colour to the liquid composition,
   - a substrate comprising one or more aldohexoses, preferably glucose,
b) providing into the liquid composition a biological sample containing one or more bacteria of the *Enterobacteriaceae* family,
c) carrying out a metabolization reaction of the one or more aldohexoses,
d) watching a colour change of the liquid composition.

The present method is adapted to the rapid detection of polymyxin susceptibility/resistance in all species of *Enterobacteriaceae* since aldohexose oxydation is a common metabolic pathway in *Enterobacteriaceae. Enterobacteriaceae* may be, for example, *Escherichia coli, Klebsiella pneumoniae,* and more particularly the species cited in the experimental section.

The liquid growth medium may be a conventional non-selective growth medium for bacteria, which does not interfere with the present method such as a Cation-Adjusted Mueller-Hinton broth. The liquid growth medium allows growth and multiplication of *Enterobacteriaceae* which produce enzymes providing metabolization of aldohexoses. Drigalski and Mac Conckey media interfere with the method.

A selective growth medium for bacteria prior to antibiotic susceptibility testing may be blood agar, chocolate agar, Luria-Bertani agar, Mueller Hinten agar, or media particularly used for screening for *Enterobacteriaceae* such as Uriselect-4, eosine- methylene blue medium.

A preferred polymyxin antibiotic compound is for example colistin sulphate or polymyxin B.

The defined concentration of polymyxin antibiotic in the liquid composition is adapted to inhibit *Enterobacteriaceae* sensitive to polymyxin antibiotics. A single polymyxin antibiotic or a mixture of polymyxin antibiotics may be used.

The pH of the liquid composition may be adjusted, for example in accordance with the pH indicator used. For example, if the pH indicator used is phenol red, the pH of the final medium may be adjusted to be about 6.6-6.7, preferably with a mineral acid such as a HCl 1M solution.

The pH indicator may be any pH indicator detecting acidification of an about neutral medium, and is for example bromothymol blue, preferably phenol red.

Since the method of the invention is based on aldohexose metabolization by Enterobacteriaceae for providing acidic compounds, any substrate comprising glucose may be used. The substrate may be an aldohexose compound, or a mixture of compounds, including for example mannose or arabinose and preferably glucose or a glucose compound. The expression "substrate comprising one or more aldohexoses" encompasses a specific aldose such as glucose per se. Preferably D(+) glucose is used as substrate comprising one or more aldohexoses. In the present invention, "aldohexose" means a C5 or C6 aldohexose.

The present method can be performed with biological samples, such as serum samples, urine samples etc particularly when an urgent assessment is necessary. The present method can also be performed with bacterial cultures resulting from any type of infection (bloodstream, urinary tract, pulmonary, device-associated infections..) and resulting from screening process (carriage in the gut flora). The method is particularly suited for severely-ill patients and patients undergoing resuscitation which are an important target of MDR bacteria.

Preparation of a culture of the enterobacteria to be tested may be implemented according to well known culture techniques.

Incubation of the enterobacteria in the above medium provides metabolization reaction of aldohexoses by *Enterobacteriaceae* and takes place under aerobic conditions. For example, the reaction may be implemented in wells of a multi well plate, such as a 96 wells plate.

Watching a colour change of the liquid composition is very easy. Detection of pH change is discernable by naked eyes by using pH indicator whose colour changes for example from red/orange to yellow in the case of phenol red. Of course colour changes may be evidenced by more complex techniques such as spectrophotometry which may allow recording the colour change.

The colour change allows determining the susceptibility or resistance to polymyxins of bacterial strains belonging to the family of *Enterobacteriaceae.* No colour change with a polymyxin antibiotic indicates lack of resistance of the strain or sensitivity of the strain to said polymyxin antibiotic: the bacterial growth was inhibited and metabolization reaction of aldohexoses did not happen. On the contrary, growth of a bacterial culture in presence of a polymyxin antibiotic provides metabolization of aldohexoses and reveals resistance of the tested strain to polymyxin antibiotic.

Watching a colour change of the liquid composition is preferably performed at a predetermined time. The colour change may be assessed for example at T=10 min (for checking absence of spontaneous colour change in the wells) and then every hour for 4 h by visual inspection. Of course other intervals, particularly shorter intervals may be implemented. According to the invention, the colour change is assessed preferably between 15 min and 4 hours, particularly between 30 min and 4 hours, more particularly between 1 h and 3 h, and very particularly approximately 2 hours after the beginning of the metabolization reaction of aldohexoses.

Preferably, for each sample or strain, two wells are inoculated in parallel, with and without polymyxin antibiotic, and their colour change may thus be easily compared.

A further object of the present invention is a diagnostic kit based on metabolization of an aldohexose substrate in presence of a polymyxin antibiotic.

More particularly a further object of the present invention is a diagnostic kit for determining susceptibility or resistance to polymyxins in *Enterobacteriaceae* comprising containers, wherein one or more containers such as wells contain(s)
- a liquid (or a solid) growth medium for *Enterobacteriaceae,*
- a polymyxin antibiotic compound,
- a pH indicator providing a colour to the liquid composition,
- a substrate comprising one or more aldohexoses, preferably glucose.

The diagnostic kit of the invention may further comprise a surfactant, such as a polysorbate, preferably polysorbate 80.

Preferably, one or more containers/wells are used as a control. Some controls preferably comprise all the ingredients of the containers used for the diagnostic, but the polymyxin antibiotic compound. When different polymyxin antibiotic compounds are used, preferably to each diagnostic container including a specific polymyxin antibiotic compound corresponds one control container.

Preferably, two wells containing two different concentrations of the tested polymyxin antibiotic compound are used for one, more than one, or all the tested polymyxin antibiotic compounds.

The one or more containers/wells are preferably provided on a plate, such as a multi well plate.

When a solid growth medium for *Enterobacteriaceae* is used, the liquid necessary for implementing the method of the invention may be provided by the sample to be tested, by a diluent such as sterile water of NaCl 0.85% or both.

A container used for the diagnostic comprises for example:
- 1 to 5 mg powder growth medium for *Enterobacteriaceae,*
- 0.5 µg to 2 µg polymyxin antibiotic compound when polymyxin is added ; no polymyxin antibiotic compound in control wells,
- 0.005 to 0.1 mg pH indicator,
- a substrate comprising 1 to 5 mg aldohexose(s), preferably glucose.

A preferred container comprises for example:
- about 3.75 mg powder for growth medium for *Enterobacteriaceae,*
- about 0.75 µg polymyxin antibiotic compound when polymxyin is added; no polymyxin antibiotic compound in control wells.
- about 7.5 µg pH indicator (phenol red)
- a substrate comprising about 1.5 mg glucose,
wherein the powder for growth medium for *Enterobacteriaceae* is Cation-Adjusted Mueller-Hinton broth powder, the polymyxin antibiotic compound is colistin sulphate or polymyxin B, the substrate comprising glucose is D (+) glucose.

In terms of concentration of the ingredients in the reactive medium composed of the liquid composition and the sample, a container used for the diagnostic comprises
- about 25 g/L solid growth medium for *Enterobacteriaceae,*
- about 5 mg/L polymyxin antibiotic compound when polymyxin is added
- about 0.05 g/L pH indicator,
- a substrate comprising about 10 g/L glucose,

Preferably, a kit comprises a series of wells containing different polymyxin antibiotic compounds.

The strain to be tested will be added at a concentration of 1 McFarland (ca. 10 ⁸CFU/ml) in 200 µl of NaCl solution (0.85%) in wells with or without polymyxin in pH adjusted solution to 6.6-6.7.

The present method offers the possibility to determine in 2-3 hours, in -vitro susceptibility or resistance to polymyxins of *Enterobacteriaceae.* Rapid determination of susceptibility to polymyxins if of utmost importance before implementing a polymyxin-based therapy whereas rapid determination of resistance to polymyxins is important to prevent the development of nosocomial outbreaks due to such resistant isolates (*K. pneumoniae* ++).

The current reference technique (broth dilution technique) is labour force - and time- consuming (18 h). Therefore, this reference technique is not performed currently in routine labs and strains have to be sent to reference labs. The method of the invention is perfectly adapted (i) to the clinical need for a personalized test to guide therapy decisions, (ii) to perform extended clinical surveys with polymyxins for treatment of MDR bacteria, (iii) to perform surveillance study at the worldwide level to know the prevalence of true and evoluting polymyxin resistance in Enterobacteriaceae, (iv) and to evaluate newer compounds under development with similar structures and properties to polymyxins. Use of the present method may also avoid overuse and blind usage of polymyxins that may be the driving force for selection of polymyxin resistance.

The method of the invention offers the possibility to screen for polymyxin susceptibility/resistance to any lab worldwide without relying on the time-consuming intervention of a reference laboratory. Combined screening of the two main polymyxin molecules used in clinical settings worldwide, i.e. colistin sulphate and polymyxin B, can be easily made concomitantly by using the method. The method is rapid, specific, sensitive, easy-to-handle and to interpret, and cost-effective since reagents and needed materials are affordable to any microbiology lab. Its results can be obtained by naked eyes; no reading device is required.

The following examples illustrate the present invention.

Preferred conditions for implementing the method described above also apply to the other subjects of the invention envisaged above such as the kits.

The scope of the invention can be understood better by referring to the examples given below, the aim of which is to explain the advantages of the invention.

### Example 1 : Polymyxin test

### 1. Preparation of standardized Enterobacteriaceae inocula

For a better comparison between the well with and without colistin sulphate, standardized *Enterobacteriaceae* inocula were prepared.

Each inoculum was prepared by mixing 1 öse (10 µl) of a fresh culture of the tested bacteria in 5 ml of sterile NaCl 0.85% (at a density of 3 Mac Farland, i.e. about 10⁹ CFU/ml).

A series of standardized saline inocula of 41 enterobacterial strains from worldwide origin (Europe, America, Africa) and from human and veterinary medicine was prepared (Table).

### 2. Preparation of reactive medium

250 ml of reactive medium was prepared by mixing the compounds hereafter:
- a broth powder of a non selective growth medium for bacteria, the Cation-Adjusted Mueller-Hinton broth (MHB-CA) : 6.25 g
- a colour indicator, the phenol red : 0.0125 g
- sterile distilled water 225 ml
- sterile D (+)-glucose (10 %) anhydrous (sterilization by filtration) : 25 ml

MHB-CA powder was from bioRad (Marnes-La-Coquette), D(+)-Glucose anhydrous 10% was from Roth (Karlsruhe) and phenol red was from Fluka (Saint-Louis, USA).

The pH of the final reactive medium was adjusted to 6.6 and 6.7 with HCl 1M.

Colistin or polymyxin B was added or not at the concentration of 5 mg/L in the reactive medium. Standard colistin sulphate and polymyxin B were from Sigma Aldrich (Saint-Louis, France). Those antibiotic molecule powders were kept at 4°C before their use, whereas polymyxin dilutions were kept at -20°C.

### 3. Preparation of containers with the reactive medium and the bacterial inocula

For each strain, two wells of a plastic sterile plate with 96 shaped wells were inoculated in parallel with and without polymyxin (colistin sulphate or polymyxin B) in order to compare their colour change.

Plastic sterile plates were from Sarstedt (Nümbrecht, Germany). The tray may be prepared as follows :
1. 150 µl of reactive medium colistin free (obtained in 1.) in the first line
2. 150 µl of reactive medium with 5 mg/l of colistin sulphate in the second line (final concentration = 3.75 mg/l)
3. 50 µl of NaCl alone in the first column (one well colistin-free and one well with colistin sulphate) (medium control)
4. 50 µl of the bacterial suspension (obtained in 2.) for the control strains and for every strain in the other column (one well colistin-free and one well with colistin)

After mixing of the bacterial suspension to the reactive medium by pipetting up and down, the final concentration of bacteria was 2.5.10⁸ CFU/ml in each well and the final selective concentration of colistin or polymyxin B in the well with polymyxin was 3.75 mg/L.

### 4. Incubation

The inoculated microdilution trays were covered and incubated for 4 h at 37°C without agitation (shaking did not provide improved results).

### 5. Reading and interpretation

The results of the tests were recorded at 10 min (checking for no spontaneous colour change in the well) and then every hour for a maximum of 4 hours by visual inspection. No colour change of the well with colistin sulphate or polymyxin B indicated susceptibility to colistin sulphate or polymyxin B and negativity of those tests. On the contrary, a colour change (orange to yellow) indicates the positivity of the test meaning growth capability (related to glucose metabolism) in presence of colistin sulphate or polymyxin B, i.e. resistance to colistin sulphate or polymyxin B.

### 6. Determination of MIC values

Determination of MIC values were performed using the reference technique (broth microdilution in Cation-Adjusted Mueller-Hinton broth) according to the CLSI guidelines. Polymyxin B and colistin (Sigma-Aldrich, Saint-Louis) were tested over a range dilution from 0.12 to 128 µg/ml and 5.10⁵ CFU/ml of bacteria in each well was used. Following the EUCAST breakpoints (http://www.eucast.org/), strains with a colistin MICs of ≤ 2 µg/ml were categorized as susceptible, although those with MICs of > 2 µg/ml were resistant.

All experiments were repeated in triplicate.

### Example 2 : Polymyxin test

A multi well plate for implementing the invention was prepared as follows:

| Well | | Composition^{a} | To add^{b} | Expected results |
|---|---|---|---|---|
| A | Medium control | X | 200 µl of NaCl 0.85 % | Orange |
| B | Colistin susceptible control | X without colistin sulphate | 200 µl of colistin-susceptible strain suspension | Yellow |
| C | Colistin susceptible control | X with colistin sulphate 0.75 µg | 200 µl of colistin-susceptible strain suspension | Orange |
| D | Colistin resistant control | X without colistin sulphate | 200 µl of colistin-resistant strain suspension | Yellow |
| E | Colistin resistant control | X with colistin sulphate 0.75 µg | 200 µl of colistin-resistant strain suspension | Yellow |
| F | Test | X without colistin sulphate | 200 µl of strain to be tested suspension | Yellow |
| G | Test | X with colistin sulphate 0.75 µg | 200 µl of strain to be tested suspension | Orange or yellow |

| | | | | |
|---|---|---|---|---|
| ^{a}X = Composition common to all wells: - Cation-Adjusted Mueller-Hinton broth powder 3.75 mg - Phenol red 7.5 µg - D (+) glucose 1.5 mg ^{b}The bacterial suspension strain was prepared in NaCl 0.85 % reaching a density of 3 Mac Farland. | | | | |

A similar polymyxin test was prepared, using polymyxin B 0.75 µg instead of colistin sulphate 0.75 µg.

Results for all tested strains are reported in Tables 1-3 hereunder.

**Table 1: Strains naturally-resistant to colistin**

| Strain | Species | Colistin MIC (mg/l) | Colistin resistance | Molecular mechanism of resistance | Results of the method of the invention |
|---|---|---|---|---|---|
| Pmir | *P. mirabilis* | >128 | + | Natural resistance | + |
| Pstu | *P. stuartii* | >128 | + | Natural resistance | + |
| Mmor | *M. morganii* | >128 | + | Natural resistance | + |
| Smar | *S*. *marcescens* | >128 | + | Natural resistance | + |

**Table 2: Strains resistant to colistin**

| Strain | Species | Colistin MIC (mg/l) | Colistin resistance | Molecular mechanism of resistance | Results of the method of the invention |
|---|---|---|---|---|---|
| C25 | *K. pneumoniae* | 64 | + | PmrA G53C | + |
| AF1b | *K. pneumoniae* | 16 | + | PmrB T157P | + |
| 75b | *K. pneumoniae* | 128 | + | PhoP D191Y | + |
| T1b | *K. pneumoniae* | 128 | + | *mgrB* gene truncated with IS5-like) | + |
| C26 | *K. pneumoniae* | 128 | + | *mgrB* gene truncated with IS | + |
| C27 | *K. pneumoniae* | >128 | + | *mgrB* Δnt23/33 (frameshift) | + |
| C23 | *K. pneumoniae* | 128 | + | Δ*mgrB* | + |
| C24 | *K. oxytoca* | 64 | + | *mgrB* promotor truncated with ISKpn26-like | + |
| 11 FR | *K. pneumoniae* | 32 | + | Unknown | + |
| 30742 | *E. coli* | 8 | + | Unknown | + |
| 27563 | *E. coli* | 16 | + | Unknown | + |
| 27837 | *E. coli* | 16 | + | Unknown | + |
| 27850 | *E. coli* | 16 | + | Unknown | + |
| 27852 | *E. coli* | 8 | + | Unknown | + |
| C28 | *E. cloacae* | 32 | + | Unknown | + |
| HM | *E. cloacae* | >128 | + | Unknown | + |
| ER | *H. alvei* | 16 | + | Unknown | + |

**Table 3: Strains susceptible to colistin**

| Strain | Species | Colistin MIC (mg/l) | Colistin resistance | Molecular mechanism of resistance | Results of the method of the invention |
|---|---|---|---|---|---|
| ATCC 25922 | *E. coli* | 0.25 | - | NA | - |
| Top10 | *E. coli* | 0.12 | - | NA | - |
| 1 FR | *K. pneumoniae* | 0.12 | - | NA | - |
| 2 FR | *K. pneumoniae* | 0.12 | - | NA | - |
| 3 FR | *E. coli* | 0.12 | - | NA | - |
| 4 FR | *K. pneumoniae* | 0.25 | - | NA | - |
| 5 FR | *E. coli* | 0.25 | - | NA | - |
| 6 FR | *E. aerogenes* | 0.12 | - | NA | - |
| 7 FR | *E. cloacae* | 0.12 | - | NA | - |
| 8 FR | *C*. *freundii* | 0.12 | - | NA | - |
| 9 FR | *K. pneumoniae* | 0.5 | - | NA | - |
| 10 FR | *E. coli* | 0.25 | - | NA | - |
| C112 | *K. pneumoniae* | 0.12 | - | NA | - |
| C115 | *K. pneumoniae* | 0.12 | - | NA | - |
| C129 | *E. cloacae* | 0.25 | - | NA | - |
| C130 | *K. pneumoniae* | 0.12 | - | NA | - |
| C132 | *K. pneumoniae* | 0.12 | - | NA | - |
| C136 | *C*. *freundii* | 0.12 | - | NA | - |
| C140 | *E. coli* | 0.12 | - | NA | - |
| C144 | *E. cloacae* | 0.25 | - | NA | - |

| | | | | | |
|---|---|---|---|---|---|
| NA: not applicable. +, yellow ; -, orange | | | | | |

Four strains were naturally resistant to colistin, twenty strains were fully susceptible to colistin, and seventeen strains had an acquired mechanism of resistance to colistin. The molecular mechanisms of colistin resistance of many of the tested colistin-resistant enterobacterial strains had been determined previously (Tables). In most of the cases, resistance to colistin/polymyxin B was associated to gene mutation or gene disruption in genes modifying the LPS structure (Tables).

A perfect correlation (100%) was obtained between colistin sulphate resistance as determined by using the reference broth dilution technique and positivity of the present method and, conversely, colistin sulphate susceptibility and negativity of the present method (Table).

Recording of the colour change of the wells shows that final results are best obtained 2 h after inoculation (most results are obtained after only 1 h of incubation).

Strains that were naturally resistant to colistin such as *Proteus mirabilis* and *Serratia marcescens* gave, as expected, a positive test result.

The present method may be adapted to single tube as well as a multi well plates such as 96-well plates for testing even a single strain in any routine laboratory. The culture media from which the bacterial inoculum is prepared may not interfere with the results of the present method since identical results were obtained when cultures were obtained from Luria Bertani, Mueller-Hinton, Uriselect-4, eosine- methylene blue medium, blood agar, and chocolate agar. However interference has been identified using culture media such as Drigalski and Mac Conckey media. Identical results were obtained by using polymyxin B instead of colistin as selective antibiotic.

Similar results were obtained by using other pH indicators, pH indicator concentrations, colistin sulphate or polymyxin B concentrations, bacterial inocula, suspension media, and aldohexose(s) concentrations.

### References

Clinical and Laboratory Standards Institute. 2014. Performance standards for antimicrobial susceptibility testing; 24th Informational supplement. Document M100-S24. Clinical and Laboratory Standards Institute, Wayne, PA.
Falagas, M. E., P. I Rafailidis, and D. K. Matthaio. 2010. Resistance to polymyxins: Mechanisms, frequency and treatment options. Drug Resist. Update. 13:132-138.
IDSA Policy Paper. 2011. The bacterial challenge: time to react. 2009, Clin Infect Dis 52: S397-S428□REF20: ECDC/EMEA Joint Technical Report.
Hindler, J.A., and R. M. Humphries. 2013. Colistin MIC variability by method for contemporary clinical isolates of multidrug-resistant Gram-negative bacilli. J. Clin. Microbiol. 51:1678-1684.
Jayol, A., L. Poirel, A. Brink, M. V. Villegas, M. Yilmaz, and P. Nordmann. 2014 Resistance to colistin associated to a single amino acid change in protein PmrB among Klebsiella pneumoniae of worldwide origin. Antimicrob. Agents Chemother 58:4762-4766.
Kieffer, N., L. Poirel, P. Nordmann, J. Y. Madec, and M. Haenni. 2015. Emergence of colistin resistance in Klebsiella pneumoniae from veterinary medicine. J. Antimicrob. Chemother, in press.
Kumar, A., P. Ellis, Y. Arabi, D. Roberts, B. Light, J.E. Parillo, P. Dodek, G. Wodd, A. D. Simon, C. Peters, M. Ashan, D. Chateau, and Cooperative Antimicrobial Therapy of Septic shock database research group. 2009. Initiation of inappropriate antimicrobial therapy results in a fivefold reduction of survival in human septic shock. Chest 136: 1237-1248
Gales, A. C., R. N Nones, and H. S. Sader. 2011. Contemporary activity of colistin and polymyxin B against a worldwide collecion of Gram-negative pathogens:results form the Sentry Antimicrobial Surveillance Program (2006-09). J. Antimicrob. Chemother. 66:2070-2074.
Garnacho-Montero, J., T. Aldabo-Pallas, C. Garnacho-Montero, A. Cayuela, R. Jimenez, S. Barroso, C. Ortiz-Leyba. 2006. Timing of adapted antibiotic therapy is a greated determinant of outcome than are TNF and IL-10 polymorphisms in patients with sepsis. Critical Care 2006 10 R111.
Humphries, R. M. 2014 Susceptibility testing of polymyxins: where are we now? Pharmacotherapy 2014. doi:10.1002/phar.1505.
Hugh, R.,and E. Leifson. 1953. The taxonomic significance of fermentative versus oxidative metabolism of carbohydrates by various gram negative bacteria. J Bacteriol 66: 24-26.
Monaco, M., T. Giani, M. Raffone, F. Arena, A. Garcia-Fernandez, S. Pollini. 2014. Colistin resistance superimposed to endemic carbapenem-resistant Klebsiella pneumonie; a rapidly evolving problem in Italy, November 2013 to April 2014. Euro Surveill. 19. pii 20939.
Nation, R. L., J. Li, O. Cars, W. Couet, M. N. Dudley, K. S. Kaye, J. W. Mouton, D. L. Paterson, V. H. Tam, U. Theuretzbacher, B. Y. Tsuji, and J. Turnidge. 2014. Framework for optimisation of the clinical use of colistin and polymyxin B, the Prato polymyxins consensus. Lancet Infect. Dis., in press.
Poirel, L. A. Jayol, S. Bontron, M.-V. Villegas, M. Ozdemar, S. Türkoglu, and P. Nordmann. 2014. The mrgB gene as a key target for acquired resistance to colistin in Klebsiella pneumoniae. J. Antimicrob. Chemother., 70:75-80.
Nordmann, P., and L. Poirel. 2014. Emerging and important resistance in Gram negatives: epidemiology, theory and practice. Rev. Med. Suisse. 10:902-907.
Nordmann, P., and L. Poirel. 2014. The difficult-to-control spread of carbapenemase-producing Enterobacteriacae. Clin. Microb. Infect, 20:821-830.

## Claims

1. A method for determining in solution susceptibility or resistance to a polymyxin antibiotic of bacteria of the *Enterobacteriaceae* family, comprising the steps consisting in
a) providing a liquid composition comprising
- a liquid growth medium for *Enterobacteriaceae,*
- a polymyxin antibiotic compound at a defined concentration adapted to inhibit *Enterobacteriaceae* sensitive to polymyxin antibiotics,
- a pH indicator providing a colour to the liquid composition,
- a substrate comprising one or more aldohexoses,
b) providing into the liquid composition a biological sample containing one or more bacteria of the *Enterobacteriaceae* family,
c) carrying out a metabolization reaction of the one or more aldohexoses,
d) watching a colour change of the liquid composition.

2. The method according to claim 1, **characterized in that** the liquid growth medium for *Enterobacteriaceae* is Cation-Adjusted Mueller-Hinton broth.

3. The method according to claim 1 or 2, wherein the polymyxin antibiotic compound is selected from the group consisting of colistin sulphate and polymyxin B.

4. The method according to claim 1, wherein the substrate comprising one or more aldohexoses is D(+) glucose.

5. The method according to claim 2, **characterized in that** the pH indicator used is phenol red and the pH of the final medium is adjusted to about 6.6-6.7.

6. The method according to any one of claims 1 to 5, **characterized in that** the method is performed with a bacterial culture resulting from an infection.

7. The method according to any one of claims 1 to 6, **characterized in that** the colour change of the liquid composition is assessed between 30 min and 4 hours after the beginning of the metabolization reaction of the one or more aldohexoses.

8. A diagnostic kit for determining susceptibility or resistance to polymyxins in *Enterobacteriaceae* comprising containers, wherein one or more containers contains
- a liquid or a solid growth medium for Enterobacteriaceae,
- a polymyxin antibiotic compound,
- a pH indicator providing a colour to the liquid composition,
- a substrate comprising one or more aldohexoses.

9. A kit according to claim 8 wherein the one or more containers further contains a surfactant.

10. A kit according to claim 8 or 9 wherein the one or more containers for determining susceptibility or resistance to polymyxins in *Enterobacteriaceae* comprises:
- about 3.75 mg Cation-Adjusted Mueller-Hinton broth powder,
- about 0.75 µg polymyxin B,
- about 7.5 µg phenol red
- a substrate comprising about 1.5 mg D (+) glucose.
